# EUROPEAN PATENT SPECIFICATION

(11) **EP 3 811 906 B1**
(45) Date of publication and mention of the grant of the patent: **04.05.2022**
(21) Application number: 20203140.7
(22) Date of filing: 21.10.2020
(51) Int. Cl.: A61F 2/46, B25F 1/00

(54) **MODULAR ORTHOPEDIC IMPLANT EXTRACTION TOOL**
OUTIL D'EXTRACTION MODULAIRE D'IMPLANTS ORTHOPÉDIQUES
MODULARES EXTRAKTIONSWERKZEUG FÜR ORTHOPÄDISCHE IMPLANTATE

(30) Priority: 21.10.2019 US 201916658606
(43) Date of publication of application: 28.04.2021
(73) Proprietor: Shukla Medical, St. Petersburg, FL 33709 (US)
(72) Inventor: SWEITZER, Zachary Robert, Keyport, NJ New Jersey 07735 (US); KEACH, Nicholas Christopher, Lutz, FL Florida 33559 (US)
(74) Representative: Cameron Intellectual Property Ltd

(56) References cited:
- US-A1- 2007 100 347
- US-A1- 2011 296 963
- US-A1- 2013 166 032
- US-A1- 2016 270 929

## Description

### BACKGROUND OF THE DISCLOSURE

Exemplary embodiments of the subject disclosure relate generally to the field of medical device implant extraction tools. Specifically, the subject disclosure relates to an orthopedic implant extraction tool having a modular jaw. The closest prior art is document US 2016/270929 A1, which defines the preamble of claim 1.

Orthopedic implant extraction tools contain jaws to grasp an orthopedic implant. Frequently, these jaws can break upon continued use, often as a result of improper or unintended use of the tool. Accordingly, there remains a need for a modular jaw that can withstand the clamping and extraction forces placed upon the orthopedic implant extraction tool, but that can be used as a consumable part and replaced, as needed, without requiring replacement of the entire tool.

### SUMMARY OF THE DISCLOSURE

The invention is defined in claim 1. One aspect of the subject disclosure provides a modular jaw for attachment to an orthopedic implant extraction tool. The modular jaw includes an elongated body, a claw about a distal end of the elongated body for engaging an object, a locking mechanism about a posterior end of the elongated body for engaging the orthopedic implant extraction tool, and a distally facing surface about a proximal end of the elongated body. The locking mechanism includes a dovetail lock and a detent mounted to the dovetail lock.

In one embodiment, the locking mechanism extends from about a midportion of the elongated body. The dovetail lock can be a tapered dovetail lock and the detent can be a ball detent. In one embodiment, the modular jaw includes a posteriorly extending tapered tenon (e.g., a curved posteriorly extending tapered tenon) about the proximal end of the elongated body. The elongated body can include a first planar posterior end and a second planar posterior end offset from the first planar posterior end, and the claw can be in the form of a posteriorly extending tapered lip.

The subject disclosure provides an orthopedic implant extraction tool that includes a handle, a gripping handle pivotably connected to the handle, and a modular stationary jaw connectable to the handle. The modular stationary jaw includes a body, a claw about a distal end of the body, and a locking mechanism for engaging the gripping handle. The locking mechanism includes a dovetail lock, a detent, and a distally facing surface about a posterior end of the body. The orthopedic implant extraction tool further includes a movable jaw connected to the gripping handle, and a shaft connectable to the handle for locking the modular stationary jaw in position.

The handle includes a cooperating locking mechanism engageable with the locking mechanism of the modular stationary jaw. The handle can include a corresponding dovetail lock about an anterior face of the handle for engaging the dovetail lock of the modular stationary jaw. The handle can further include a fastener about a posteriorly facing end of the handle.

In one embodiment, the shaft has an overall diameter that overlaps with a proximal end of the modular stationary jaw when connected to the handle. In certain embodiments, the shaft directly engages the modular stationary jaw when connected to the handle.

### BRIEF DESCRIPTION OF THE SEVERAL VIEWS OF THE DRAWINGS

The following detailed description of an exemplary embodiment of the subject disclosure will be better understood when read in conjunction with the appended drawings. For the purpose of illustrating the present disclosure, there is shown in the drawings an exemplary embodiment. It should be understood, however, that the subject application is not limited to the precise arrangements and instrumentalities shown.
FIG. 1 is a perspective view of a modular jaw in accordance with an exemplary embodiment of the subject disclosure;
FIG. 2 is a left side view of the modular jaw of FIG. 1;
FIG. 3 is a right side view of the modular jaw of FIG. 1;
FIG. 4 is a bottom plan view of the modular jaw of FIG. 1;
FIG. 5 is a top plan view of the modular jaw of FIG. 1;
FIG. 6 is a front view of the modular jaw of FIG. 1;
FIG. 7 is a rear view of the modular jaw of FIG. 1;
FIG. 8 is a perspective view of an orthopedic implant extraction tool that includes the modular jaw of FIG. 1;
FIG. 9 is another perspective view of the orthopedic implant extraction tool of FIG. 8, shown with certain components omitted or rendered transparent for purposes of clarity;
FIG. 10 is an enlarged partial perspective view of the orthopedic implant extraction tool of FIG. 8, shown with certain components omitted for purposes of clarity; and
FIG. 11 is another enlarged partial perspective view of the orthopedic implant extraction tool of FIG. 8, shown with certain components omitted for purposes of clarity.
FIG. 12 is a perspective view of a shaft of the orthopedic implant extraction tool of FIG. 8.

### DETAILED DESCRIPTION

Reference will now be made in detail to an exemplary embodiment of the subject disclosure illustrated in the accompanying drawings. Wherever possible, the same or like reference numbers will be used throughout the drawings to refer to the same or like features. It should be noted that the drawings are in simplified form and are not drawn to precise scale. In reference to the disclosure herein, for purposes of convenience and clarity only, directional terms such as upper, lower, top, bottom, above, below and diagonal, are used with respect to the accompanying drawings. Such directional terms used in conjunction with the following description of the drawings should not be construed to limit the scope of the subject disclosure in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1 %, or ±0.1% from the specified value, as such variations are appropriate.

"Substantially" as used herein shall mean considerable in extent, largely but not wholly that which is specified, or an appropriate variation therefrom as is acceptable within the field of art.

Throughout the subject application, various aspects thereof can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Furthermore, the described features, advantages and characteristics of the exemplary embodiments of the subject disclosure may be combined in any suitable manner in one or more embodiments. One skilled in the relevant art will recognize, in light of the description herein, that the subject disclosure can be practiced without one or more of the specific features or advantages of a particular exemplary embodiment. In other instances, additional features and advantages may be recognized in certain embodiments that may not be present in all exemplary embodiments of the present disclosure.

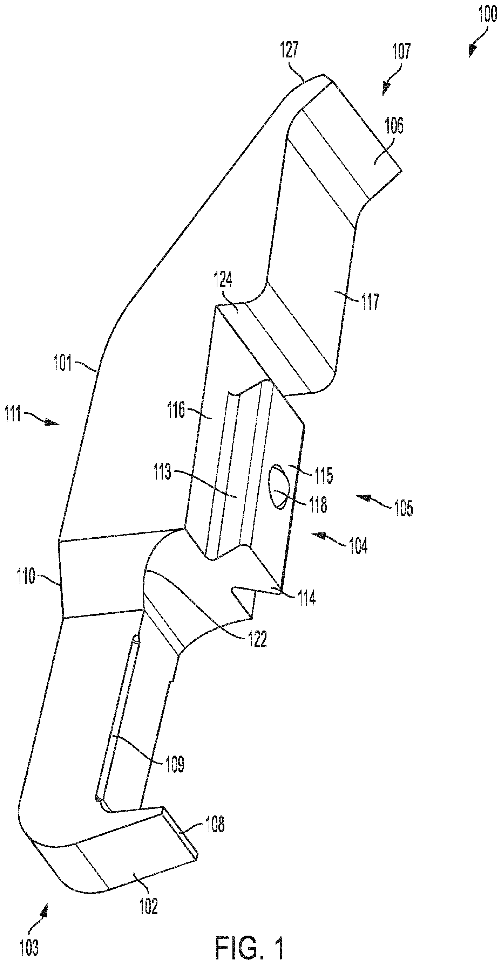

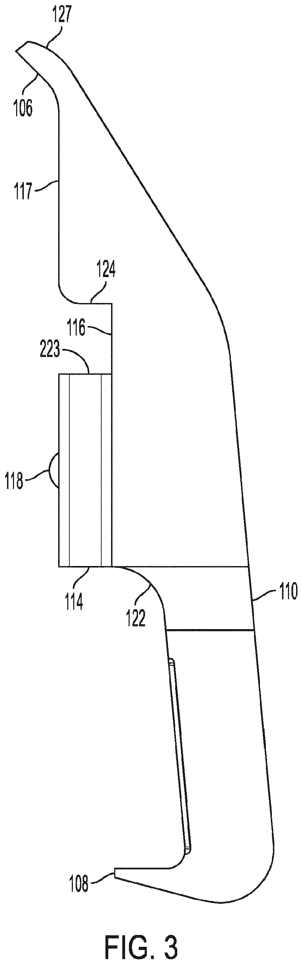

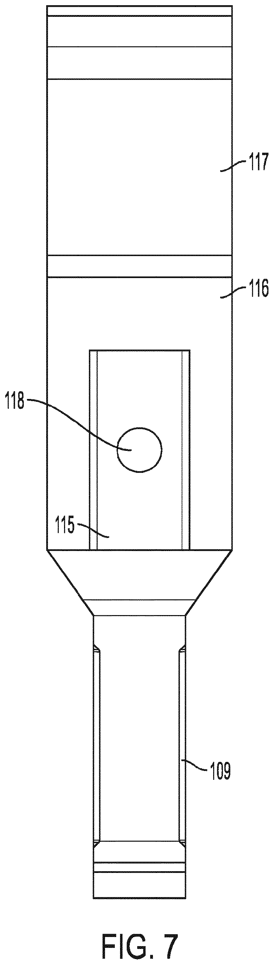

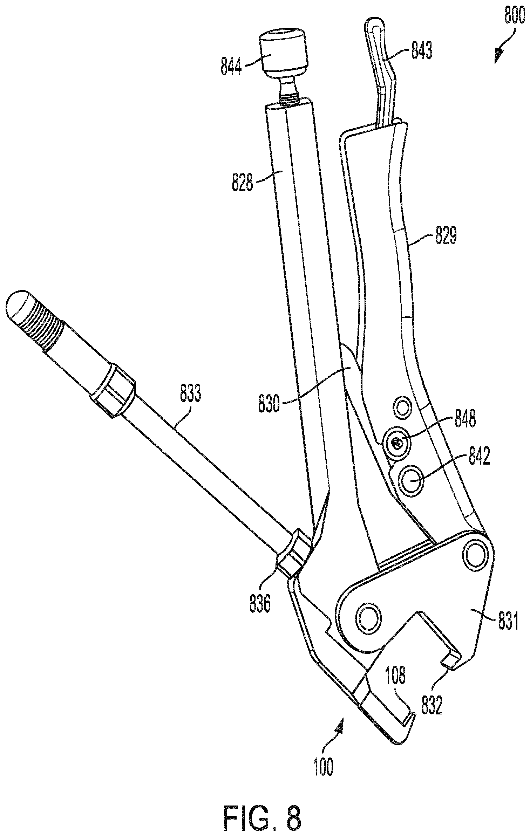

Referring now to the drawings, FIG. 1-7 illustrate a modular jaw 100 for attachment to an orthopedic implant extraction tool, such as, for example, the orthopedic implant extraction tool 800 shown in FIG. 8. The modular jaw 100 includes an elongated body 101, a claw 102 about a distal end 103 of the elongated body, and a locking mechanism 104 about a posterior end 105 of the elongated body for engaging the orthopedic implant extraction tool. The modular jaw further includes a distally facing surface 106 about a proximal end 107 of the elongated body.

The distal end 103 includes the claw 102 that is configured, e.g., as a lip for engaging an object such as an orthopedic implant, such as a tibial orthopedic implant (tibial tray) used in knee replacement surgery. The lip 108 can, in certain exemplary embodiments, be in the form of a sharp edge to penetrate the interface of the orthopedic implant and bone, which can include implant-bone cement or an implant-bone boundary. As shown in FIG. 1, the lip 108 is a posteriorly extending tapered lip that is slightly tapered inward from the furthest distal reach of the distal end 103.

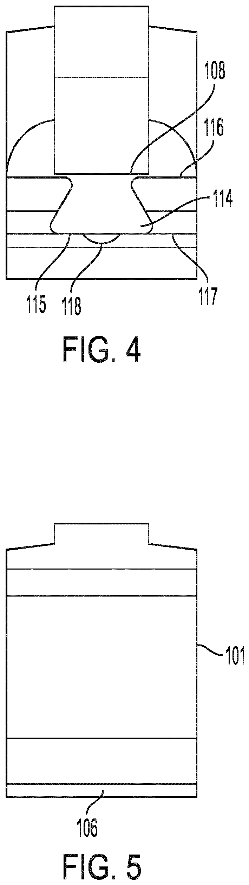

As best shown in FIG. 4, the lip 108 defines a posterior end of the claw 102 that terminates about a first planar posterior end 116. Alternatively, in other exemplary embodiments, a longer lip can be provided that extends to or before a second planar posterior end 117 that is offset from the first planar posterior end 116.

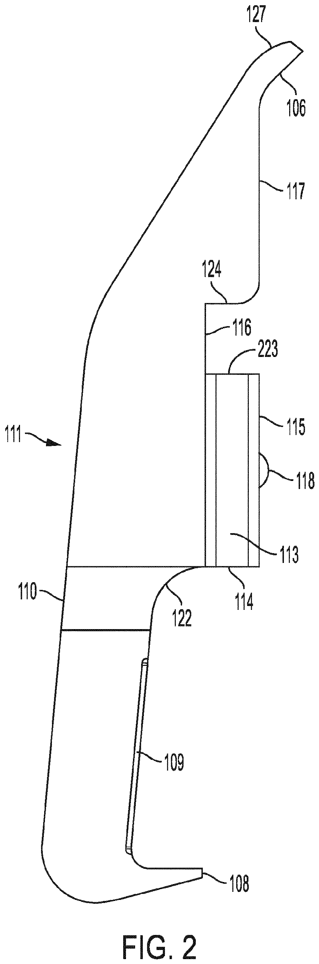

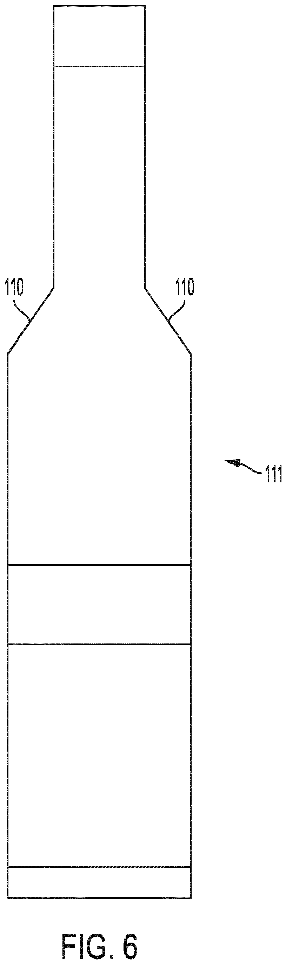

The elongated body 101 proceeds from the distal end 103 with a width that is the same, or substantially similar to, the width of the lip 108. The edges along the posterior end 105 of this section are provided in this exemplary embodiment with relived edges 109. The elongated body 101 then flares outwardly at flares 110 toward the proximal end to provide a second, larger width at along a midportion 111 of the elongated body, this second, larger width continuing for the remainder of the elongated body, as best shown in FIG. 6. Posterior flares 122 similarly extend outward to provide the second, larger width, but also extend posteriorly to provide an increased width, as best shown in FIG. 2. The posterior flares 122 terminates about the plane defined by the first planar posterior end 116. For example, the second, larger width can be 1.5, 2, 2.5 or 3 times the width of the lip 108, though other dimensioning could be provided.

About the posterior end 105 of the elongated body, a locking mechanism 104 is provided. The locking mechanism extends from the midportion 111 of the elongated body 101. In this particular embodiment, the locking mechanism includes a dovetail lock 113, which tapers to provide an increasing width as one proceeds posteriorly (from left to right as oriented in FIG. 1), reaching a maximum at a posterior dovetail lock plane 115. The dovetail lock 113 is provided with a pair of faces, distal face 114 and proximal face 223 that are generally trapezoidal shaped.

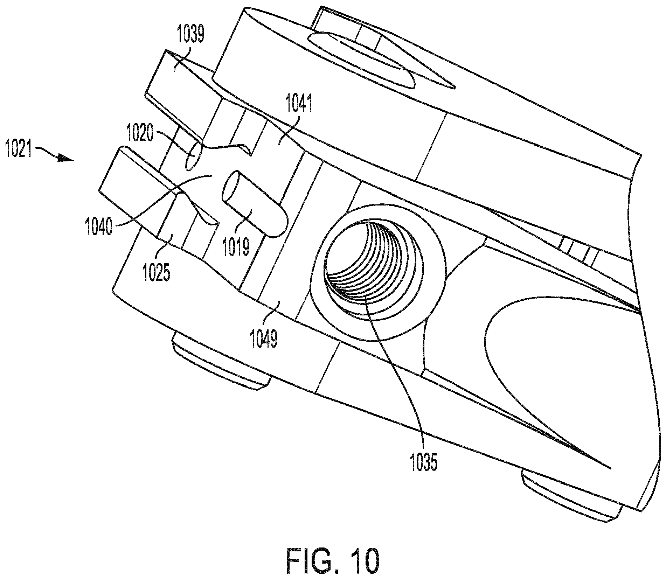

The dovetail lock 113 includes the posterior dovetail lock plane 115 that is generally parallel to the first planar posterior end 116 from which the dovetail lock 113 originates. As best shown in FIG. 4, the dovetail lock plane 115 is along the same plane as second planar posterior end 117 and has a width substantially equal to the width of the lip 108. As will be discussed in connection with FIG. 10 below, the locking mechanism 104 is shaped to engage with a corresponding locking mechanism 1021 that can be located on an anterior face 1041 of a handle to the orthopedic implant extraction tool.

Detent 118 is mounted on the posterior dovetail lock plane 115 of the dovetail lock 113. In this particular embodiment, the detent 118 is a ball detent that is provided with a ball biased outwardly by a compression spring (not shown). The detent 118 is mounted about a central portion of the dovetail lock, where it can engage components of the corresponding locking mechanism 1021, particularly, a ball detent track 1019 and a ball detent seat 1020 located on the anterior face 1041 of the handle 828, as best shown in FIG. 10 and discussed below. Detent 118 serves to hold the modular jaw 100 in place during assembly, as discussed below.

The dovetail lock 113 terminates to form the proximal face 223 of the dovetail lock. A posteriorly extending stop 124 is located axially with respect to the distal face 114 and the proximal face 223 of the dovetail lock. In this embodiment, the stop 124 forms a face that is dimensioned to engage a complementarily shaped surface of the orthopedic implant extraction tool, such as, for example, stop surfaces 1025 shown in FIG. 10. The stop 124, when engaged with the stop surface 1025, prevents further movement of the modular jaw 100 in the distal direction when the modular jaw is assembled onto the orthopedic implant extraction tool, and also positions the detent 118 over the detent seat 1020 of the orthopedic implant extraction tool.

In this particular embodiment, the posteriorly extending stop 124 provides the offset between the first planar posterior end 116 and the second planar posterior end 117. As best shown in FIGS. 2-3, the first planar posterior end 116 is parallel to, or substantially parallel to, the second planar posterior end 117, with the stop 124 oriented perpendicular to both the first planar posterior end and the second planar posterior end.

The proximal end 107 of the elongated body 101 tapers along the portion of the jaw defined by the second planar posterior end 117. The second planar posterior end 117 meets the distally facing surface 106 that is also provided about the elongated body 101. The proximal end 107 continues to taper along the portion defined by the distally facing surface 106 to define a posteriorly extending tapered tenon 127 about the proximal end 107 of the elongated body (and about the posterior end 105). The distally facing surface 106 is provided at an end of the posteriorly extending tapered tenon 127, as best shown in FIG. 1. This distally facing surface 106 is provided about the proximal end 107 and the posterior end 105 of the elongated body 101.

The modular jaw 100 is attachable to the orthopedic implant extraction tool 800 shown in FIGS. 8 - 11. Tool 800 includes a handle 828 that is connectable to the modular jaw 100, and a gripping handle 829 coupled to the handle 828 via a locking link 830. The gripping handle 829 is pivotably connected to the handle 828 via movable jaw 831, which in turn is provided with a second claw, e.g., in the form of a lip 832 that, like lip 108 of the modular jaw 100, is sized and shaped to grasp an orthopedic implant such as, for example, a tibial implant tray used in knee replacement surgery.

A shaft 833 is connected to the handle 828 via fastener 934 located about a posteriorly facing end 937 of the handle. Though not limited thereto, the shaft 833 can be, for example, a shaft to an extractor extension, such as a connecting rod or an extraction rod.

In this particular embodiment, the fastener 934 is a threaded bore. The shaft 833 can include a threading 1251 about a distal end of the shaft with threads shaped to engage with the threads 1035 of the threaded bore. The shaft 833 can further include a head 836, or the head 836 can be provided by a nut included as part of the shaft 833. In either scenario, the head 836 and/or shaft 833 defines an overall diameter of the shaft in the particular embodiment disclosed in FIG. 8. The head 836 is optional, and not present in certain embodiments, in which case the shaft defines the overall diameter.

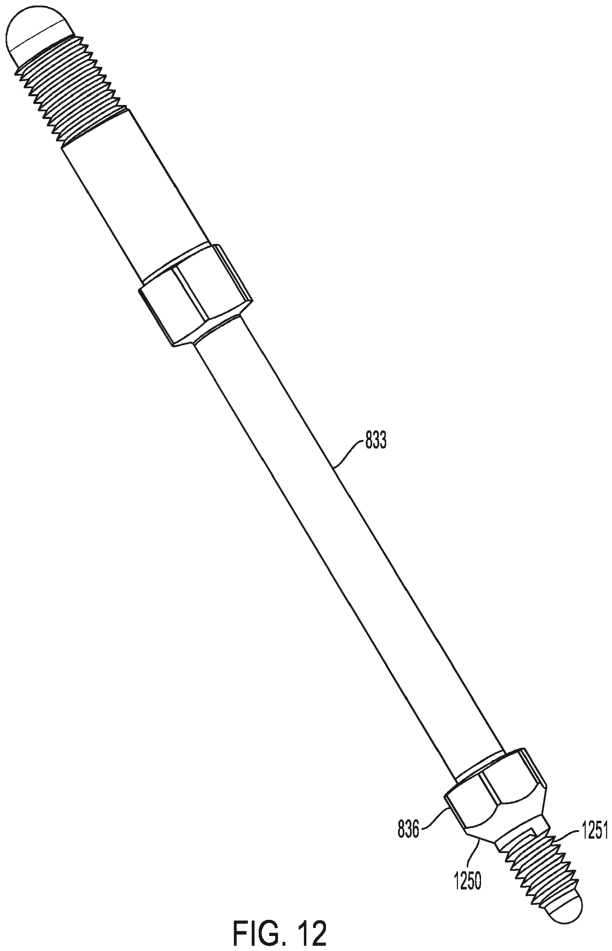

The shaft 833 can be provided with a larger diameter as it exits handle 828, as compared to the diameter of the shaft at the threading 1251 that internally engages the fastener 934 (e.g., a threaded bore). As shown best in FIG. 12, the shaft 833 can include, for example, a flare 1250 that provides a progressively larger diameter proceeding from the threading 1251 to the head 836 of the shaft. Alternatively, for example, a beveled nut could be employed to provide the overall diameter of the shaft 833. Other configurations can be employed so that the shaft provides an overall diameter that overlaps with a proximal end of the modular stationary jaw when connected to the handle 828.

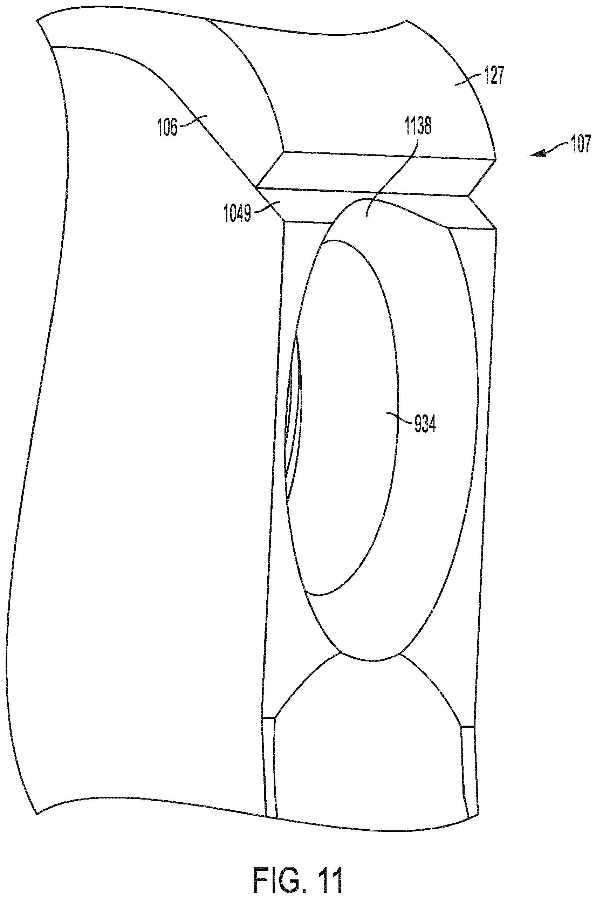

As shown best in FIG. 11, the fastener 934 is provided with beveled edges 1138 to accommodate and seat the larger diameter of the shaft (e.g. at the flare 1250). When the shaft 833 is connected to the handle 828 via the fastener 934, a part of the shaft directly contacts the proximal end of the modular jaw 100.

With reference to FIG. 10, the handle 828 includes a corresponding locking mechanism 1021 which corresponds to the locking mechanism 104. In this particular embodiment, the locking mechanism 124 is a male locking mechanism and the corresponding locking mechanism 1021 is a female locking system. The corresponding locking mechanism 1021 of the handle 828 includes, in this particular embodiment, a corresponding dovetail lock 1039 provided about the anterior face 1041 of the handle. The corresponding dovetail lock 1039 contains a passageway 1040 shaped to receive the distal face 114 of the dovetail lock 113. The width of the passageway 1040 decreases as one proceeds anteriorly (from bottom to top as oriented in FIG. 10) to correspond to the increasing width of the dovetail lock 113. This dovetail engagement provides a secure engagement of the modular jaw 100 in response to a clamping force.

The anterior face 1041 further includes a ball detent track 1019 and a ball detent seat 1020. The ball detent track 1019 is axially aligned with the ball detent seat 1020, both of which being axially aligned with stop 124, and are each sized to receive the ball to the detent 118. The ball detent track 1019 serves to allow the modular jaw to be easily slid into the handle 828. A sloped face 1049 is provided proximal to the anterior face 1041 and provides a proximally faced surface, as shown in FIG. 10. Sloped face 1049 is shaped complementary to the distally facing surface 106 to directly engage the distally facing surface 106 when the modular jaw is secured onto the handle.

Orthopedic implant extraction tool 800 can include other standard components, such as those components disclosed in the orthopedic implant extraction tools disclosed in U.S. Patent No. 10,085,852 which is hereby incorporated by reference in its entirety.

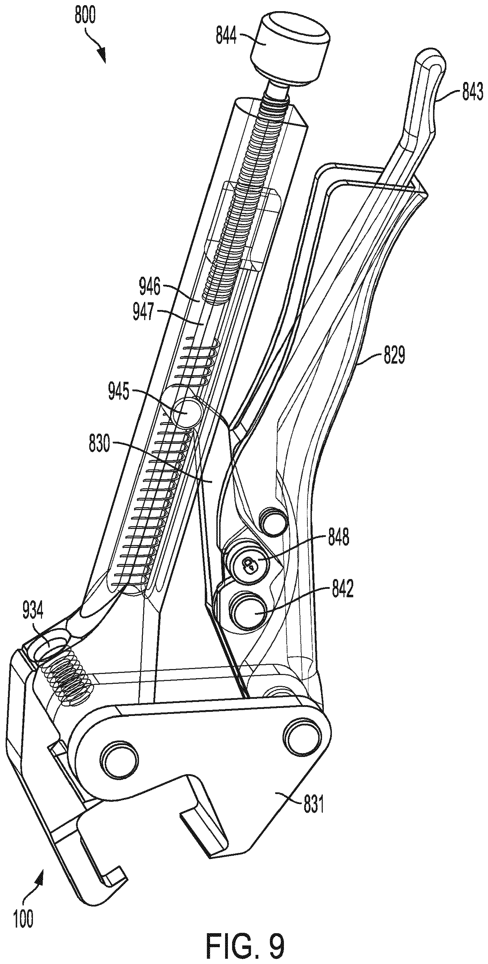

For example, as best shown in FIG. 9, in which the handle 828 and the gripping handle 829 is rendered transparent solely for purposes of illustration, the locking link 830 has one end mounted for rotation about pin 842 in the gripping handle 829 with the other end 945 mounted within a longitudinal groove 946 that is located within the handle 829. A lock 843, secured within the gripping handle 829, can be manually manipulated such that it contacts the locking link 830 to prevent the lips 108 and 832 from closing past the adjusted opening provided by a thumb screw 844. Thumb screw 844 is connected to a shaft 947 that engages end 945 of the locking link 830 and serves to adjustably limit the range of movement of the locking link 830, which in turn serves to limit the extent to which the lips 108 and 832 can be closed. A locking pin 848 is provided that is connected to a sliding rod (not shown) such that, when the locking pin 848 is depressed, moves the sliding rod to engage the locking link 830 to prevent movement of the locking link and lock the handles in their current position. These components of the tool 800 are provided for purposes of illustration only. The modular jaw 100 can be used with other tools having different designs and is not limited to use with the orthopedic implant extraction tool 800 described herein.

In operation, the distal face 114 of the dovetail lock 113 of the modular jaw 100 is slid into the passageway 1040 of the corresponding dovetail lock 1039. This engagement is aided placing the ball from detent 118 into the ball detent track 1019. When the stop 124 of the modular jaw engages the stop surface 1025, the ball to the detent is seated in ball detent seat 1020 and the modular jaw 100 is fully engaged with the handle 828. As noted, a compression spring (not shown) biases the ball against ball detent seat 1020.

When the modular jaw 100 is fully engaged with the handle 828, shaft 833 is engaged with fastener 934 by screwing the shaft 833 into the fastener 934 via threading 1251. In this particular embodiment, the fastener 934 is a threaded bore. Doing so causes the shaft 833 to directly contact and engage the proximal end 107 of the modular jaw, which in turn renders the tool 800 ready for use. More particularly, direct contact of the shaft with the proximal end 107 of the modular jaw secures distally facing surface 106 against the sloped face 1049 of the handle to lock the modular jaw into place so that the modular jaw is secure under all ranges of motion. To remove the modular jaw 100, the shaft 833 is removed from the handle 828, and then the modular jaw can be slid out from the handle in the opposite direction from which it was engaged.

It will be appreciated by those skilled in the art that changes could be made to the exemplary embodiments described above without departing from the broad inventive concept thereof. It is to be understood, therefore, that the invention is not limited to the particular embodiments disclosed, but it is intended to cover modifications within the scope of the claims defined herein.

## Claims

1. An orthopedic implant extraction tool (800) comprising:
a handle (828); a
gripping handle (829) pivotably connected to the handle; a movable jaw (831) connected to the gripping handle; and
a modular jaw (100) adapted to be connected to the handle, the modular jaw including:
an elongated body (101),
a claw (102) about a distal end (103) of the elongated body for engaging an object, and
a locking mechanism (104) about a posterior end (105) of the elongated body for engaging the orthopedic implant extraction tool, **characterised in that** the locking mechanism includes:
a dovetail lock (113),
a detent (118) mounted to the dovetail lock, and a distally facing surface (106) about a proximal end (107) of the elongated body; wherein the orthopedic implant extraction tool further comprises
a shaft (833) adapted to be connected to the handle for locking the modular jaw in position.

2. The orthopedic implant extraction tool of claim 1, wherein the locking mechanism (104) of the modular jaw (100) extends from about a midportion (111) of the elongated body (101).

3. The orthopedic implant extraction tool of any one of claims 1 or 2, wherein the dovetail lock (113) of the locking mechanism (104) of the modular jaw (100) is a tapered dovetail lock.

4. The orthopedic implant extraction tool of any one of claims 1-3, wherein the detent (118) of the locking mechanism (104) of the modular jaw (100) is a ball detent.

5. The orthopedic implant extraction tool of any one of claims 1-4, further comprising a posteriorly extending tapered tenon (127) about the proximal end (107) of the elongated body (101) of the modular jaw (100).

6. The orthopedic implant extraction tool of claim 5, wherein the posteriorly extending tapered tenon (127) is curved.

7. The orthopedic implant extraction tool of any one of claims 1-6, wherein the elongated body (101) of the modular jaw (100) includes a first planar posterior end (116) and a second planar posterior end (117) offset from the first planar posterior end.

8. The orthopedic implant extraction tool of claim 7, wherein the claw (102) of the modular jaw (100) includes a posterior end that terminates about a plane defined by one of the first or second planar posterior ends (116, 117).

9. The orthopedic implant extraction tool of any one of claims 1-8, wherein the claw (102) of the modular jaw (100) is a posteriorly extending tapered lip (108).

10. The orthopedic implant extraction tool of any one of claims 1-9, wherein the
handle (828) includes a cooperating locking mechanism (1021) adapted to engage with the locking mechanism (104) of the modular jaw (100).

11. The orthopedic implant extraction tool of any one of claims 1-10, wherein the handle (828) includes a corresponding dovetail lock (1021) about an anterior face (1041) of the handle (828) for engaging the dovetail lock (113) of the modular jaw (100).

12. The orthopedic implant extraction tool of any one of claims 1-11, wherein the shaft (833) has an overall diameter that overlaps with a proximal end of the modular jaw (100) when connected to the handle (828).

13. The orthopedic implant extraction tool of any one of claims 1-12, wherein the shaft (833) directly engages the modular jaw (100) when connected to the handle (828).

14. The orthopedic implant extraction tool of any one of claims 1-13, wherein the modular jaw (100) is a modular stationary jaw.

15. The orthopedic implant extraction tool of claim 14, wherein the handle (828) includes a fastener (934) about a posteriorly facing end (937) of the handle.

## Patentansprüche

1. Extraktionswerkzeug (800) für ein orthopädisches Implantat, umfassend:
einen Griff (828);
einen Haltegriff (829), der schwenkbar mit dem Griff verbunden ist;
eine bewegliche Backe (831), die mit dem Haltegriff verbunden ist; und
eine modulare Backe (101), die angepasst ist, um mit dem Griff verbunden zu werden, wobei die modulare Backe Folgendes beinhaltet:
einen länglichen Körper (101),
eine Klaue (102) um ein distales Ende (103) des länglichen Körpers zum Einführen eines Gegenstandes, und
einen Verriegelungsmechanismus (104) um ein hinteres Ende (105) des länglichen Körpers zum Einführen des Extraktionswerkzeugs für das orthopädische Implantat, **dadurch gekennzeichnet, dass** der Verriegelungsmechanismus Folgendes beinhaltet:
eine Schwalbenschwanz-Verriegelung (113),
eine Raste (118), die auf der Schwalbenschwanz-Verriegelung montiert ist, und
eine distal zugewandte Oberfläche (106) um ein proximales Ende (107) des länglichen Körpers;
wobei das Extraktionswerkzeug für das orthopädische Implantat weiter einen Schaft (833) umfasst, der angepasst ist, um mit dem Griff zum Verriegeln der modularen Backe in Position verbunden zu werden.

2. Extraktionswerkzeug für ein orthopädisches Implantat nach Anspruch 1, wobei sich der Verriegelungsmechanismus (104) der modularen Backe (100) von etwa einer Mittenposition (111) des länglichen Körpers (101) aus erstreckt.

3. Extraktionswerkzeug für ein orthopädisches Implantat nach einem der Ansprüche 1 oder 2, wobei die Schwalbenschwanz-Verriegelung (113) des Verriegelungsmechanismus (104) der modularen Backe (100) eine sich verjüngende Schwalbenschwanz-Verriegelung ist.

4. Extraktionswerkzeug für ein orthopädisches Implantat nach einem der Ansprüche 1-3, wobei die Arretierung (118) des Verriegelungsmechanismus (104) der modularen Backe (100) eine Kugelraste ist.

5. Extraktionswerkzeug für ein orthopädisches Implantat nach einem der Ansprüche 1-4, weiter einen sich nach hinten erstreckenden sich verjüngenden Zapfen (127) um das proximale Ende (107) des länglichen Körpers (101) der modularen Backe (100) umfassend.

6. Extraktionswerkzeug für ein orthopädisches Implantat nach Anspruch 5, wobei der sich nach hinten erstreckende sich verjüngende Zapfen (127) gekrümmt ist.

7. Extraktionswerkzeug für ein orthopädisches Implantat nach einem der Ansprüche 1-6, wobei der längliche Körper (101) der modularen Backe (100) ein erstes ebenes hinteres Ende (116) und ein zweites ebenes hinteres Ende (117), das von dem ersten ebenen hinteren Ende versetzt ist, beinhaltet.

8. Extraktionswerkzeug für ein orthopädisches Implantat nach Anspruch 7, wobei die Klaue (102) der modularen Backe (100) ein hinteres Ende beinhaltet, das um eine Ebene abschließt, die durch eines von dem ersten oder zweiten ebenen hinteren Ende (116, 117) definiert wird.

9. Extraktionswerkzeug für ein orthopädisches Implantat nach einem der Ansprüche 1-8, wobei die Klaue (102) der modularen Backe (100) eine sich nach hinten erstreckende sich verjüngende Lippe (108) ist.

10. Extraktionswerkzeug für ein orthopädisches Implantat nach einem der Ansprüche 1-9, wobei der Griff (828) einen zusammenwirkenden Verriegelungsmechanismus (1021) beinhaltet, der angepasst ist, um in den Verriegelungsmechanismus (104) der modularen Backe (100) einzugreifen.

11. Extraktionswerkzeug für ein orthopädisches Implantat nach einem der Ansprüche 1-10, wobei der Griff (828) eine entsprechende Schwalbenschwanz-Verriegelung (1021) um eine Innenseite (1041) des Griffs (828) zum Eingreifen in die Schwalbenschwanz-Verriegelung (113) der modularen Backe (100) beinhaltet.

12. Extraktionswerkzeug für ein orthopädisches Implantat nach einem der Ansprüche 1-11, wobei der Schaft (833) einen Gesamtdurchmesser aufweist, der sich bei Verbindung mit dem Griff (828) mit einem proximalen Ende der modularen Backe (100) überschneidet.

13. Extraktionswerkzeug für ein orthopädisches Implantat nach einem der Ansprüche 1-12, wobei der Schaft (833) bei Verbindung mit dem Griff (828) direkt in die modulare Backe (100) eingreift.

14. Extraktionswerkzeug für ein orthopädisches Implantat nach einem der Ansprüche 1-13, wobei die modulare Backe (100) eine stationäre modulare Backe ist.

15. Extraktionswerkzeug für ein orthopädisches Implantat nach Anspruch 14, wobei der Griff (828) einen Verschluss (934) um ein nach hinten zugewandtes Ende (937) des Griffs beinhaltet.

## Revendications

1. Outil d'extraction d'implant orthopédique (800) comprenant :
une poignée (828) ;
une poignée de préhension (829) reliée de manière pivotante à la poignée ;
une mâchoire mobile (831) reliée à la poignée de préhension ; et
une mâchoire modulaire (100) conçue pour être reliée à la poignée, la mâchoire modulaire incluant :
un corps allongé (101),
une griffe (102) autour d'une extrémité distale (103) du corps allongé pour venir en prise avec un objet, et
un mécanisme de verrouillage (104) autour d'une extrémité arrière (105) du corps allongé pour venir en prise avec l'outil d'extraction d'implant orthopédique, **caractérisé en ce que** le mécanisme de verrouillage inclut :
un élément de verrouillage en queue d'aronde (113),
un cran (118) monté sur l'élément de verrouillage en queue d'aronde, et
une surface orientée de manière distale (106) autour d'une extrémité proximale (107) du corps allongé ;
dans lequel l'outil d'extraction d'implant orthopédique comprend en outre une tige (833) conçue pour être reliée à la poignée pour verrouiller la mâchoire modulaire en position.

2. Outil d'extraction d'implant orthopédique selon la revendication 1, dans lequel le mécanisme de verrouillage (104) de la mâchoire modulaire (100) s'étend approximativement depuis une partie intermédiaire (111) du corps allongé (101).

3. Outil d'extraction d'implant orthopédique selon l'une quelconque des revendications 1 ou 2, dans lequel l'élément de verrouillage en queue d'aronde (113) du mécanisme de verrouillage (104) de la mâchoire modulaire (100) est un élément de verrouillage en queue d'aronde effilé.

4. Outil d'extraction d'implant orthopédique selon l'une quelconque des revendications 1-3, dans lequel le cran (118) du mécanisme de verrouillage (104) de la mâchoire modulaire (100) est un cran à bille.

5. Outil d'extraction d'implant orthopédique selon l'une quelconque des revendications 1-4, comprenant en outre un tenon effilé s'étendant vers l'arrière (127) autour de l'extrémité proximale (107) du corps allongé (101) de la mâchoire modulaire (100).

6. Outil d'extraction d'implant orthopédique selon la revendication 5, dans lequel le tenon effilé s'étendant vers l'arrière (127) est incurvé.

7. Outil d'extraction d'implant orthopédique selon l'une quelconque des revendications 1-6, dans lequel le corps allongé (101) de la mâchoire modulaire (100) inclut une première extrémité arrière plane (116) et une seconde extrémité arrière plane (117) décalée par rapport à la première extrémité arrière plane.

8. Outil d'extraction d'implant orthopédique selon la revendication 7, dans lequel la griffe (102) de la mâchoire modulaire (100) inclut une extrémité arrière qui prend fin autour d'un plan défini par l'une de la première ou de la seconde extrémité arrière plane (116, 117).

9. Outil d'extraction d'implant orthopédique selon l'une quelconque des revendications 1-8, dans lequel la griffe (102) de la mâchoire modulaire (100) est une lèvre effilée s'étendant vers l'arrière (108).

10. Outil d'extraction d'implant orthopédique selon l'une quelconque des revendications 1-9, dans lequel la poignée (828) inclut un mécanisme de verrouillage coopérant (1021) conçu pour venir en prise avec le mécanisme de verrouillage (104) de la mâchoire modulaire (100).

11. Outil d'extraction d'implant orthopédique selon l'une quelconque des revendications 1-10, dans lequel la poignée (828) inclut un élément de verrouillage en queue d'aronde correspondant (1021) autour d'une face avant (1041) de la poignée (828) pour venir en prise avec l'élément de verrouillage en queue d'aronde (113) de la mâchoire modulaire (100).

12. Outil d'extraction d'implant orthopédique selon l'une quelconque des revendications 1-11, dans lequel la tige (833) présente un diamètre global qui empiète sur une extrémité proximale de la mâchoire modulaire (100) lorsqu'elle est reliée à la poignée (828).

13. Outil d'extraction d'implant orthopédique selon l'une quelconque des revendications 1-12, dans lequel la tige (833) vient directement en prise avec la mâchoire modulaire (100) lorsqu'elle est reliée à la poignée (828).

14. Outil d'extraction d'implant orthopédique selon l'une quelconque des revendications 1-13, dans lequel la mâchoire modulaire (100) est une mâchoire modulaire fixe.

15. Outil d'extraction d'implant orthopédique selon la revendication 14, dans lequel la poignée (828) inclut un élément de fixation (934) autour d'une extrémité orientée vers l'arrière (937) de la poignée.
